# EUROPÄISCHE PATENTSCHRIFT

(11) **EP 1 631 672 B1**
(45) Veröffentlichungstag und Bekanntmachung des Hinweises auf die Patenterteilung: **20.09.2006**
(21) Anmeldenummer: 03817243.3
(22) Anmeldetag: 10.06.2003
(51) Int. Cl.: C12N 15/85, C12N 15/66, A61K 48/00

(54) **ZIRKULÄRES EXPRESSIONSKONSTRUKT FÜR GENTHERAPEUTISCHE ANWENDUNGEN**
CIRCULAR EXPRESSION CONSTRUCT FOR GENE THERAPEUTIC APPLICATIONS
PRODUIT DE SYNTHESE D'EXPRESSION CIRCULAIRE DESTINE A DES APPLICATIONS DE THERAPIE GENIQUE

(43) Veröffentlichungstag der Anmeldung: 08.03.2006
(73) Patentinhaber: Mologen AG, 14195 Berlin (DE)
(72) Erfinder: SCHROFF, Matthias, 14057 Berlin (DE); SMITH, Colin, 14057 Berlin (DE)
(74) Vertreter: Boeckh, Tobias
(86) Internationale Anmeldenummer: PCT/DE2003/001970
(87) Internationale Veröffentlichungsnummer: WO 2004/111247

(56) Entgegenhaltungen:
- EP-A- 0 967 274
- WO-A-94/09127
- WO-A-96/05297
- WO-A-98/21322
- US-A- 6 143 530
- LOPEZ-FUERTES L ET AL: "DNA vaccination with linear minimalistic (MIDGE) vectors confers protection against Leishmania major infection in mice" VACCINE, BUTTERWORTH SCIENTIFIC. GUILDFORD, GB, Bd. 21, Nr. 3-4, 13. Dezember 2002 (2002-12-13), Seiten 247-257, XP004394510 ISSN: 0264-410X
- SCHIRMBECK R ET AL: "Priming of immune responses to hepatitis B surface antigen with minimal DNA expression constructs modified with a nuclear localization signal peptide" JOURNAL OF MOLECULAR MEDICINE, SPRINGER VERLAG, DE, Bd. 79, Nr. 5-6, Juni 2001 (2001-06), Seiten 343-350, XP002252260 ISSN: 0946-2716 in der Anmeldung erwähnt
- JOHANSSON P ET AL: "PCR-generated linear DNA fragments utilized as a hantavirus DNA vaccine" VACCINE, BUTTERWORTH SCIENTIFIC. GUILDFORD, GB, Bd. 20, Nr. 27-28, 10. September 2002 (2002-09-10), Seiten 3379-3388, XP004378533 ISSN: 0264-410X
- GURUNATHAN S ET AL: "DNA vaccines: immunology, application, and optimization*." ANNUAL REVIEW OF IMMUNOLOGY. UNITED STATES 2000, Bd. 18, 2000, Seiten 927-974, XP009019110 ISSN: 0732-0582

## Beschreibung

Die Erfindung betrifft sowohl ein Herstellungsverfahren für ein minimalistisches Expressionskonstrukt aus einem zirkulären, ringförmig geschlossen DNA-Doppelstrang als auch ein entsprechendes DNA-Expressionskonstrukt. Derartige Expressionskonstrukte (Vektoren) sollen insbesondere auf dem Gebiet der Gentherapie eingesetzt werden. Unter Gentherapie soll die Einschleusung eines oder mehrerer Fremdgene in den Organismus mit therapeutischem Nutzen für das Individuum verstanden werden.

Die Gentherapie ist vor allem von der Entwicklung ungefährlicher und leicht anwendbarer in vivo Gentransfermethoden abhängig, die entweder eine effiziente und stabile Genexpression in bestimmten Zielorganen oder die gezielte Hemmung der Protein-Expression spezifischer Gene erlauben.

Aufgrund der Vielzahl der Barrieren (Plasmamembran, Endosom, Zellkern) beim Transfer von genetischem Material in die Zelle ist die Transfektion von DNA ein seltener und schlecht vorherzusagender Vorgang. So ist die unzureichende Transfektionseffizienz der bisher entwickelten Vektoren ein großes Problem, da bei der Injektion von DNA ins Gewebe ein Großteil der Zellen im Zielgewebe nicht transfiziert wird. Erfolgreich transfizierte Zellen exprimieren die transfizierten DNA-Sequenzen zudem unterschiedlich stark.

Grundsätzlich ist zwischen viralen und nicht-viralen Gentransfermethoden zu unterscheiden. Virale Transfermethoden benutzen genetisch modifizierte Viren als Transportvehikel für genetisches Material. Da Wildtyp-Viren und die von ihnen abgeleiteten Vektoren in der Regel neben einer hohen Transfektionseffizienz auch eine gute Gewebespezifität zeigen, werden sie zur Zeit am häufigsten für den Gentransfer eingesetzt.

Allerdings ist ihr Einsatz von nicht zu unterschätzenden Sicherheitsrisiken begleitet, die ihrer Verwendung in der Gentherapie massiv entgegenstehen. So stellt die Möglichkeit der Rekombination der eingeschleusten viralen Bestandteile mit im Patienten natürlich vorkommenden Viren ein inhärentes Sicherheitsrisiko dar. Aus dieser unkontrollierten Rekombination von vermehrungsfähigen und nicht-vermehrungsfähigen Viren, können neue, pathogene Hybridviren hervorgehen. Ebenso sind immunologische Reaktionen, die durch die Anti-Vektor-Immunität hervorgerufen werden, eine schwerwiegende Begleiterscheinung beim Einsatz viraler Vektoren. So führte die Anwendung einer hohen Dosis eines Adenovirus bei einem klinischen Versuch zum Tod des Patienten, dessen Ursache offensichtlich eine starke Überreaktion des Immunsystems war (Lehrman, 1999, Nature 401: 517-518). Die Fälle der Leukämieerkrankung nach Gentherapie mit viralen Gentransfersystemen zeigen, das die Probleme auch heute noch nicht gelöst sind und stellen einen schweren Rückschritt in der Gentherapie dar (Buckley RH, 2002, Lancet 360: 1185-6).

Diese Nachteile werden von den nicht-viralen Gentransfersystemen, die in der Regel von Plasmiden abgeleitet sind und auch als "nackte" DNA bezeichnet werden, vermieden. Allerdings weisen diese neben weitaus geringeren Transfektionsraten und den damit einhergehenden geringeren Expressionsraten der transferierten Sequenzen, auch eine fehlende Zell- oder Gewebespezifität auf.

Ein weiterer Nachteil bekannter, nicht-viraler Gentransfersysteme ist der relativ große Anteil an bakteriellen DNA-Sequenzen, welche in den Plasmiden enthalten sind. Diese verursachen im Zielorganismus erhebliche Probleme. So führen die natürlicherweise im Plasmid vorhandenen immunstimulatorischen Sequenzen ("ISS", z.B. nichtmethylierte Cytosin-Guanin Dinukleotide, "CpG") zur Stimulierung der Effektorzellen des Immunsystems und damit zur Ausschüttung von inflammatorischen Zytokinen und Interferonen (Krieg, 2002, Annu Rev Immunol 20:709-60). In allen Fällen, bei denen die Erzeugung eines immunologischen Th1-Phänotyps nicht gewollt oder gar kontraindiziert ist, sollte dies vermieden werden. Zu solchen Fällen gehören alle Erkrankungen mit Autoimmun-Komponenten, wie der systemische Lupus Erythematosus oder Morbus Crohn sowie die Vielzahl an Indikationen für die Gentherapie, die vollkommen ohne Beteiligung des Immunsystems ablaufen, wie die Stoffwechselerkrankung Mukoviszidose oder der Alpha-1-Antitrypsin Mangel.

Des weiteren enthalten die schon bekannten Plasmide Antibiotika-Resistenzgene, die zu ihrer Selektion notwendig sind. Die Folge der Möglichkeit einer Rekombination mit ubiquitär im Organismus vorkommenden Bakterien, birgt die Gefahr des zunehmenden Auftretens antibiotikaresistenter Bakterien in sich. Diese Verbreitung von Antibiotikaresistenzen ist in Hinblick auf die wiederholt notwendigen Applikationen der therapeutischen Gene ein schwerwiegendes Problem und daher nicht vertretbar.

Coutelle et al. gelang es durch Reduktion der bakteriellen DNA-Sequenzen in Plasmiden, sog. Minizirkel-DNA herzustellen, die in in vitro Versuchen gegenüber herkömmlichen Plasmiden zu bis zu zehnfach höheren Expressionsraten führten (Coutelle et al., 2001, J of Biol. Chem. 25: 23018-23027). Dabei wurden Plasmide verwendet, auf denen zwei Erkennungssequenzen für eine Rekombinase enthalten waren. Da diese eingefügten Rekombinationsorte bakteriellen Ursprungs sind, sind auch in dieser Art von Vektor Reste bakterieller DNA enthalten, so dass die Nachteile herkömmlicher Plasmide zwar reduziert, jedoch noch vorhanden sind. Ein zielgerichteter Gentransfer ist durch die Minizirkel ebenfalls nicht möglich, da eine spezifische und damit kontrollierbare Bindung transfervermittelnder Liganden durch die Struktur der Minizirkel nicht erreichbar ist.

Das US Patent 6,143,530 hat ebenfalls Plasmide mit einem verminderten Anteil an DNA bakteriellen Ursprungs zum Inhalt. Jedoch verläuft auch der dortige Herstellungsprozess mit Hilfe von bakteriellen Rekombinasen und bedingt so die Anwesenheit bakterieller DNA-Sequenzen, die zu vektor-induzierten, inflammatorischen Vorgängen führen können.

In dem US Patent 6,265,218 wird ein Verfahren zur Herstellung eines Vektors ohne Selektionsmarker-Gen beschrieben. Die nach dem beanspruchten Verfahren hergestellten Vektoren sollen in der Gentherapie oder zur Herstellung von pharmazeutischen Medikamenten für die Gentherapie verwendet werden. Die in dieser Druckschrift einzig ausführbaren Vektoren werden unter Verwendung eines Rekombinase-Systems hergestellt. Dies bedingt zwangsläufig Expressionskonstrukte, die vektorielle Sequenzen enthalten.

Eine andere Art von nicht-viralen Vektoren stellen minimalistische, nur teilweise doppelsträngige geschlossene Expressionskonstrukte dar. Die Darstellung derartiger Expressionskonstrukte mit linearer, kovalent geschlossener Topologie ist in der EP 0 941 318 B1 gezeigt. Diese hantelförmigen Konstrukte tragen nur die zur Expression des Zielgens absolut notwendige Sequenzinformation und vermeiden somit die Nachteile von Plasmiden mit bakteriellen Sequenzen. Ein Nachteil der hantelförmigen Expressionskonstrukte ist, dass sie im Vergleich zu Plasmiden aus zirkulär geschlossenen DNA-Doppelsträngen eine geringere Expression an therapeutischen Proteinen induzieren.

Zur Induktion einer Immunantwort, wie sie bei der prophylaktischen oder therapeutischen Impfung erwünscht ist, sind diese linearen Vektoren den konventionellen Plasmiden jedoch deutlich überlegen, da sie zu einer qualitativ und quantitativ signifikant verbesserten Immunantwort führen (Lutz et al., 2000, J Virol: 74(22):10447-57):

Zur Steigerung der Transfektionseffizienz ist der ligandenvermittelte Gentransfer bekannt. An die Vektoren werden transfervermittelnde Liganden, wie beispielsweise das Kemlokalisationssignal (NLS, Aminosäuresequenz PKKKRKV) aus dem SV-40 Virus oder das Transaktivatorprotein TAT des HIV-1 Virus, gebunden, um den Eintritt durch die Zellmembran und später in den Zellkern, zu forcieren. Damit wird versucht, die viralen Mechanismen des erfolgreichen Eindringens in Zellen zu imitieren und nicht-virale Vektoren mit effizienten Zielfindungssystemen auszustatten.

So konnte ein 10- bis 15-fach erhöhter Antikörpertiter bei einem Immunisierungsversuch nach Kopplung des NLS-Peptids an eine für das HBsAg (Hepatitis small surface Antigen) kodierende lineare Expressionskassette, im Vergleich zu einer ungekoppelten Expressionskassette, nachgewiesen werden (Schirmbeck et al., J. Mol Med. 2001 Jun;79 (5-6):343-50). Nachteilhafterweise ist auch hier die Expressionsrate im Vergleich zu den Vektoren des Standes der Technik deutlich niedriger.

Zusammenfassend sei hervorgehoben, dass trotz langjähriger und intensiver Forschung keine DNA-Vektoren entwickelt werden konnten, die eine hohe Transfektionseffizienz aufweisen und zugleich ohne Sicherheitsbedenken in der Gentherapie oder genetischen Vakzinierung von Mensch und Tier eingesetzt werden können.

Ausgehend von diesem Stand der Technik ist es Aufgabe der vorliegenden Erfindung ein Verfahren zur Verfügung zu stellen, durch das nicht-virale Expressionskonstrukte mit einer hohen Transfer- und Expressionseffizienz hergestellt werden können; ferner ein entsprechendes Expressionskonstrukt zu schaffen und Verwendungsmöglichkeiten desselben aufzuzeigen. Dabei sollen Nachteile der bekannten viralen Expressionssysteme, die im Rahmen der Gentherapie eingesetzt werden, vermieden werden.

Die vorliegende Erfindung löst dieses technische Problem durch ein Verfahren zur Herstellung eines Vektor gemäß Anspruch 1 oder Anspruch 2, also durch ein zirkuläres, von bakterieller und viraler DNA freies ringförmiges (zirkuläres) DNA-Expressionskonstrukt, das Zellen effektiv und gezielt transfizieren kann.

Unter einem zirkulären Expressionskonstrukt wird im Sinne der Erfindung ein DNA-Vektor verstanden, der lediglich aus einem zirkulären DNA-Doppelstrang besteht. Die doppelsträngige DNA besteht zumindest aus wenigstens einer Expressionskassette, wobei sich die Expressionskassette aus wenigstens einem Promotor, wenigstens der kodierenden Sequenz und gegebenenfalls wenigstens einer Polyadenylierungssequenz zusammensetzt. Zweckmäßigerweise besitzt der Vektor zum Schutz gegen E-xonukleaseabbau keine freien Enden, sondern ist ringförmig geschlossen.

In einer weiteren Ausführungsform nach Anspruch 2 ist der Vektor unter Verwendung eines Oligodesoxynukleotid kovalent geschlossen, welches die kohäsiven Fragment-Enden der ausgeschnittenen Expressionskassette kovalent schließt.

Dieses Oligodesoxyribonukleotid kann ein oder mehrere modifizierte Basen aufweisen, die die Kopplung mit einem oder mehreren Liganden ermöglichen.

In Abhängigkeit der Sequenz des verwendeten Oligodesoxyribonukleotids können sich diskrete Strukturmotive ausbilden, die eine freie und damit bessere und effizientere Zugänglichkeit zum Liganden ermöglichen. Unter Liganden sollen in diesem Zusammenhang auch Peptide, Proteine und/oder andere organische Moleküle, beispielsweise Zucker- oder Steroidmoleküle, verstanden werden, die kovalent an Vektoren gebunden zur zielgerichteten und effektiven Transfektion der Zellen führen.

Unter Transfektion soll das Einbringen von Nukleinsäuresequenzen mittels biologischer, chemischer oder physikalischer Methoden in die Zelle verstanden werden, in dessen Folge es zu anhaltender oder vorübergehender Expression von durch diese Sequenzen kodierten Proteinen oder katalytisch wirksamen RNA-Transkripten in der transfizierten Zelle kommt. Es können aber auch sogenannte "anti-sense"-Konstrukte eingeschleust werden, welche durch Hybridisierung mit der komplementären messenger-RNA die Translation und so die Protein-Expression unterbinden.

Durch das erfindungsgemäße Verfahren werden zirkuläre sowie ligandenmodifizierte zirkuläre Genexpressionskonstrukte aus einem durchgängigen DNA-Doppelstrang hergestellt. Da die Expressionskassetten dieser erfindungsgemäßen Expressionskonstrukte auf die für die Expression des therapeutischen Gens absolut notwendigen Steuermechanismen beschränkt sind, befinden sich nur genau definierte Nukleotide in der Sequenz der Vektoren, welche weder bakteriellen noch viralen Ursprungs sind. Das reduziert die Vektorgröße um 2-3 Kilobasen und geht mit der Verringerung des Gehalts an CpG-Sequenzen um ca. 90% einher. Die nach dem erfindungsgemäßen Verfahren hergestellten Expressionskonstrukte sind biologisch nicht amplifizierbar, d.h. dass sie können weder in pro- noch in eukaryontischen Zellen vermehrt werden.

Erfindungsgemäße zirkuläre Expressionskonstrukte werden durch Ausschneiden mit einer Restriktionsendonuklease des Typs IIS, bevorzugt Eco 31I, aus einem dafür geeigneten Plasmid, welches die zu exprimierenden Gensequenzen enthält, isoliert. Das entstandene kohäsive Fragment, welches die Expressionskassette enthält, wird dann durch Ligation ringförmig geschlossen. In einer Ausführungsform sind ein oder mehrere Liganden an ein ODN gekoppelt, welches die kohäsiven Fragment-Enden der ausgeschnittenen Expressionskassette schließt. Zur Herstellung dieser peptidgekoppelten Vektoren werden an das ODN zuvor durch kovalente Bindung ein oder mehrere transfervermittelnde Liganden gekoppelt. Das ODN ist durch eine oder mehrere alkylische Carbonsäure-, Amin-, Thiol-, oder Aldehydfunktionen chemisch modifizierbar.

Das Plasmidrückrat, welches in einem sekundären Restriktionsverdau durch eine Restriktionsendonuklease geschnitten wird, für die keine Erkennungssequenz in der Expressionskassette vorliegt, wird enzymatisch durch die Exonukleasefunktion der T7-DNA-Polymerase abgebaut und das verbliebene zirkuläre-doppelsträngig geschlossene erfindungsgemäße DNA-Expressionskonstrukt wird durch einen chromatographisehen Schritt aufgereinigt. Zusätzlich kann das Expressionskonstrukt noch durch Isopropanolfällung aufgereinigt werden. Durch dieses erfindungsgemäße Verfahren werden alle für die Plasmidherstellung benötigten Sequenzelemente, so auch bakterielle oder virale Sequenzen, aus dem Expressionskonstrukt entfernt.

Durch die Reduktion auf die zur Expression notwendigen Sequenzinformationen ist es möglich, den erfindungsgemäßen Vektor in einer neuartigen Kleinheit herzustellen. So ist die Herstellung dieser Art von Vektoren in Größenbereichen von 200-10000bp, bevorzugt 1000-2500bp, möglich. Im Vergleich dazu beträgt die durchschnittliche Größe eines herkömmlichen Plasmides ohne kodierende Gensequenzen 2000-3000bp und kann herstellungsbedingt in den meisten Fällen nicht unterschritten werden.

Die kovalente Kopplung der Liganden an das ODN erfolgt über ein Verbindungsmolekül und stellt dadurch eine definierte chemische Bindung dar. Es ist von Vorteil, dass die Liganden an definierten Positionen an den DNA-Vektor ligiert werden können. Ein möglicher Funktionsverlust des Promotors oder der funktionellen Gene durch Bindung der Liganden innerhalb eines funktionell sensitiven Bereichs der Sequenz ist somit ausgeschlossen. Ebenso ist es vorteilhafterweise möglich, mehrere voneinander unabhängige, jeweils definiert platzierte Liganden an den Expressionsvektor zu ligieren.

Das vorliegende erfindungsgemäße Verfahren kann auch zur einfachen gewerblichen Anwendung in Form eines Kits zur Herstellung des erfindungsgemäßen Expressionskonstruktes vorliegen. Ein derartiger Kit umfasst
(a) ein Plasmid, welches
   - die erforderlichen Erkennungssequenzen für die Restriktionsenzyme enthält, und
   - zur Amplifikation der Expressionskassette geeignet ist,
(b) einen ersten Enzymmix bestehend aus Restriktionsenzym und Ligase,
(c) einen zweiten Enzymmix bestehend aus Restriktionsenzym und Polymerase,
(d) ein im wesentlichen bekanntes Mittel zur Aufreinigung des Expressionskonstrukts,
(e) übliche Reaktionsmedien, wie Puffer, ATP, DTT, Wasser etc.
(f) einen Kontrollvektor zum Funktionsnachweis der Bestandteile des Kits.

Voraussetzung für einen derartigen Kit ist das Vorliegen einer kodierenden (beliebigen) Sequenz, die mit üblichen Methoden in das Plasmid (a) kloniert wird.

Weitere Vorteile der Erfindung sind in den übrigen Ansprüchen, der Beschreibung und den Figuren beschrieben. Die überraschende Wirkung des erfindungsgemäßen zirkulären, ligandmodifizierten Vektors aus einem DNA-Doppelstrang sowie das erfindungsgemäße Verfahren wird anhand der Figuren und den Ausführungsbeispielen deutlich; es zeigt:
- **Fig.1**: die schematische Darstellung des Herstellungsprozesses der zirkulären erfindungsgemäßen DNA-Vektoren; worin bedeuten
a) Plasmid
b) Eco 31I
c) ligand-modifizierter Oligonukleotid-Linker
d) Expressionskassette
e) bakterielle Rest-DNA
f) Eco 311
g) Gemisch aus bakterieller Rest-DNA (e) und Produkt (i)
h) T7 DNA-Polymerase
i) Produkt
- **Fig. 2**: den funktionellen Aufbau der erfindungsgemäßen peptidgekoppelten DNA-Vektoren, worin bedeuten
a) Sequenz des zu exprimierenden Genes
b) Promotorbereich
c) ligand-modifizierter Oligonukleotid-Linker
d) Poly(A)-Signal
- **Fig. 3**: den *in vitro* Expressionsvergleich von erfindungsgemäßem Vektor, herkömmlichem Plasmid und einem linearen Vektor anhand der Firefly-Luciferase Expression nach Transfektion von K562 Zellen, gemessen in rlu (relative Lichteinheiten). Dabei bedeuten:
Plasmid Plasmid pMOK, kodierend für Luciferase,
lin Vektor linearer Vektor, kodierend für Luciferase
zirk Vektor2NLS zirkulärer, erfindungsgemäßer mit 2 NLS-Peptiden gekoppelter Vektor
- **Fig. 4**: den *in vitro* Expressionsvergleich von erfindungsgemäßem Vektor, herkömmlichem Plasmid und einem linearen Vektor anhand der Firefly-Luciferase Expression nach Transfektion von Hela Zellen, gemessen in rlu (relative Lichteinheiten). Dabei bedeuten:
Plasmid Plasmid pMOK, kodierend für Luciferase,
lin Vektor linearer Vektor, kodierend für Luciferase
zirk Vektor zirkulärer, erfindungsgemäßer Vektor ohne Peptidkopplung
- **Fig. 5**: *in vivo* Expression Lac-Z kodierender zirkulärer und linearer Vektoren Dabei bedeuten:
lin Vektor linearer Vektor, kodierend für Lac-Z
lin Vektor-NLS linearer, NLS-Peptid gekoppelter Vektor, kodierend für Lac-Z
zirk Vektor-NLS zirkulärer erfindungsgemäßer, NLS-Peptid gekoppelter Vektor, kodierend für Lac-Z
- **Fig. 6**: Interferon-gamma Sekretion stimulierter Splenozyten in Mäusen Dabei bedeuten:
lin Vektor-NLS linearer, mit NLS-Peptid gekoppelter Vektor, kodierend für HBsAg
zirk Vektor-NLS zirkulärer, mit NLS-Peptid gekoppelter erfindungsgemäßer Vektor, kodierend für HBsAg

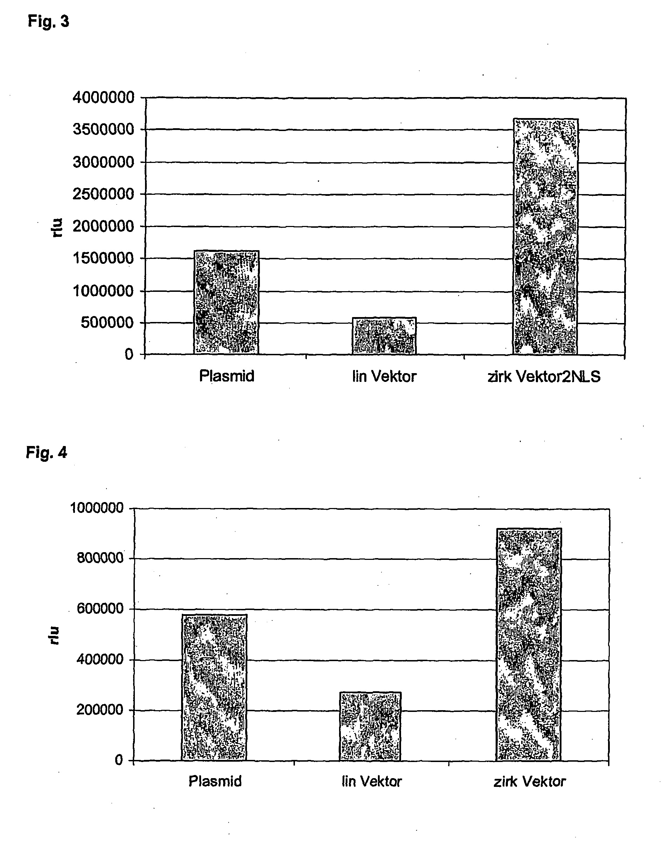

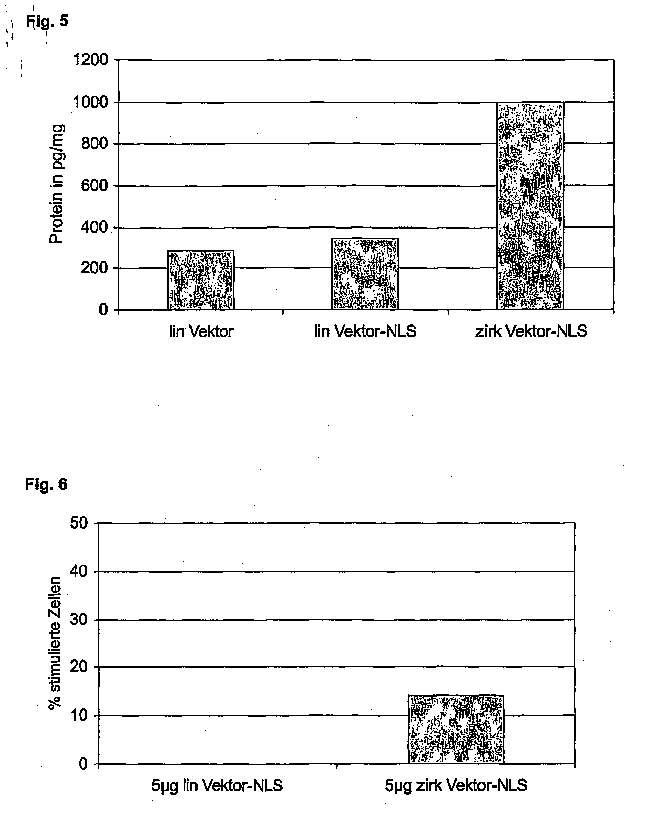

Der Expressionsnachweis mit Hilfe des Reportergens Luciferase erfolgte in vitro in zwei humanen Zelllinien. Zum Vergleich der Expressionsraten wurden für Luciferase kodierendes Plasmid, ein herkömmlicher linearer Vektor und ein erfindungsgemäßer Vektor mit und ohne Peptidkopplung eingesetzt. Überraschenderweise wird durch das beanspruchte Konstrukt mit der Peptidmodifikation NLS (Kernlokalisationssignal Aminosäuresequenz PKKKRKV aus dem SV-40 Virus) eine mehr als doppelt so hohe Expressionsrate im Vergleich mit herkömmlichem Plasmid erreicht (siehe Fig. 3). Ebenso zeigt der erfindungsgemäße zirkuläre Vektor ohne Peptidmodifikation einen deutlichen Expressionsvorteil gegenüber den Vektoren des Standes der Technik in Fig. 4.

Für *in vivo* Studien wurde Mäusen entweder ein erfindungsgemäßer oder ein herkömmlicher für das Lac-Z Gen kodierender Vektor appliziert. Anhand der quantitativen β-Galactosidase Expression fand ein Vergleich der vektorinduzierten Expression von β-Galaktosidase statt. Auch hier ist der erfindungsgemäße Vektor (als "zirk Vektor-NLS" bezeichnet) deutlich von Vorteil. Vergleichbar mit den *in vitro* Ergebnissen in Fig. 3 u. 4 wird auch *in vivo* die Expressionsrate durch die beanspruchten Vektoren um mehr als 50% gesteigert (siehe Fig. 5).

Ein weiterer *in vivo* Versuch diente dazu, die immunologischen Abläufe nach der Impfung mit linearen und erfindungsgemäßen Vektoren zu untersuchen. Dazu wurden Mäuse mit für HBsAg kodierenden Vektoren geimpft und das dadurch hervorgerufene Zytokinprofil anhand der Interferon-gamma (IFN-gamma) Ausschüttung verglichen. Interferon-gamma spielt eine Schlüsselrolle in der Immunantwort und der anti-viralen Abwehr. Die erfindungsgemäßen Vektoren sind gemäß Fig. 6 in der Lage antigenspezifische IFN-gamma sekretierende Splenozyten zu induzieren, während die linearen Vektoren bei der eingesetzten geringen Menge von 5µg an Vektor-DNA zu keiner IFN-gamma Ausschüttung führen. Für die Impfung oder Immuntherapie verschiedener Krankheiten scheint die Erzeugung einer Th1-typischen Immunreaktion vorteilhaft, besonders gilt dies für intrazelluläre Parasiten wie Leishmania und Malaria, sowie virale Erkrankungen wie HIV.

Neben den Vorteilen der signifikanten Steigerung der Genexpression und der Erzeugung einer verstärkt zellulär vermittelten Immunantwort, besitzt diese neue Art von Vektoren keine Markergene oder kodierende Sequenzen viralen oder bakteriellen Ursprungs, sondern besteht nur aus Sequenzen, die direkt für die Expression der therapeutischen Gene notwendig sind, und bietet dadurch die größtmögliche Sicherheit für den Patienten. Zum Einen werden unerwünschte immunologische oder entzündliche Prozesse vermieden, wie sie durch bakterielle oder virale DNA hervorgerufen werden, zum Anderen scheint die damit einhergehende Verringerung der Vektorgröße vorteilhafterweise zu einer erhöhten Transferrate in den Zellkern zu führen.

### Beispiel 1: Herstellung der zirkulären Vektoren

Das Plasmid pMOK-Luc wurde mit dem Restriktionsenzym Eco31I für 2h bei 37°C vollständig verdaut. Durch den Restriktionsverdau wurden zwei DNA-Fragmente erzeugt. Das eine bestand aus dem Kanamycin-Resistenzgen, sowie anderen zur Propagierung des Plasmides in Bakterien notwendigen Sequenzen, das andere Fragment bestand aus den Sequenzen, die Bestandteil des erfindungsgemäßen Vektors werden sollten, nämlich CMV-Promotor, der zu exprimierenden Gensequenz und der Polyadenylierungssequenz aus SV-40. Durch das Enzym T4-DNA-Ligase (in Ligasepuffer: 400mM Tris-HCL, 100mM MgCL₂, 5mM ATP) wurden die durch Eco31I erzeugten komplementären Enden des vektorbildenden DNA Fragments über Nacht bei 4°C miteinander ligiert. Das resultierende Gemisch an Nukleinsäuren wurde mit dem Enzym Eco 1471 behandelt. Zum Abbau der Rest-DNA vektoriellen Ursprungs wurde das Enzym T7 DNA Polymerase dem Gemisch hinzugegeben. Die verbliebene zirkuläre Expressionskassette wurde durch Anionenaustauschchromatographie aufgereinigt und mit Isopropanol gefällt.

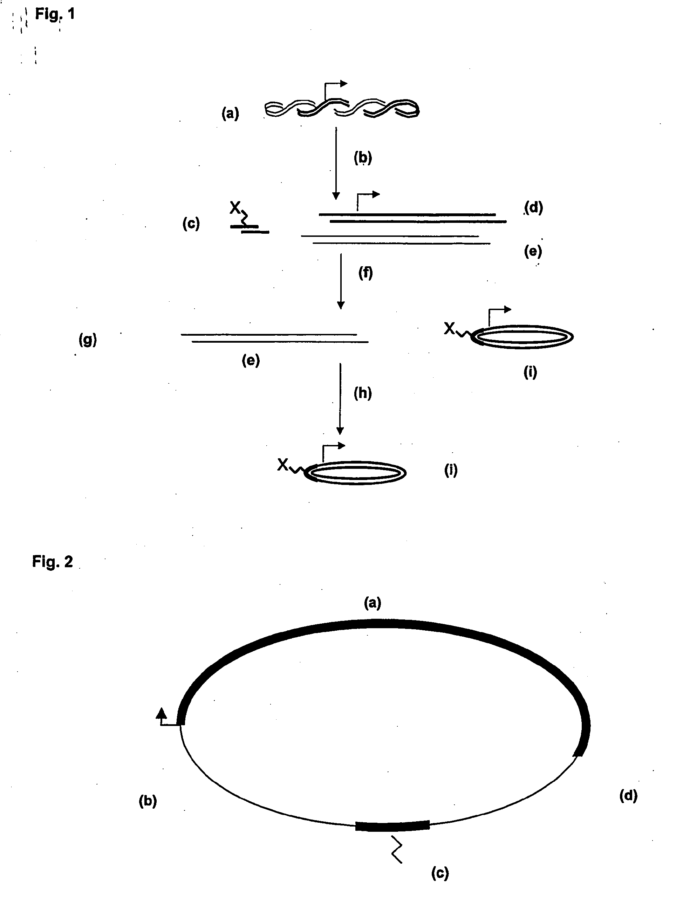

Figur 1 zeigt insoweit die schematische Darstellung des Herstellungsprozesses der zirkulären erfindungsgemäßen DNA-Vektoren: Durch den Verdau des Plasmids (a) mit Eco 31I (b) werden die Fragmente bakterielle Rest-DNA (e) und Expressionskassette (d) erzeugt. Mit Hilfe der Enzyms T4-DNA Ligase (f) und in Anwesenheit von Eco 31I (f) wird der ligand-modifizierte Oligonukleotid-Linker (c) mit (d) ligiert. Dieses Gemisch aus bakterieller Rest-DNA (g) und Produkt (i) bestehend, wird im letzten Schritt (h) mit T7 DNA-Polymerase behandelt, und führt zum Produkt (i).

### Beispiel 2a:Ligandenkopplung

Zirkuläre Expressionskassetten mit Peptid-Kopplung wurden wie folgt konstruiert: Das NLS Peptid PKKKRKV (Polin-Lysin-Lysin-Lysin-Arginin-Lysin-Valin) wurde in zwei Schritten an wahlweise ein oder beide Oligonukleotide gekoppelt. Zuerst wurde das modifizierte Oligonukleotid (5,-PH-d(GGGAACCTTCAGTxAGCAATGG bzw 5,-PH-d AGGGCCATTGCTxACTGAAGG, wobei xT für amminomodifizierte Thyminbase mit C2 Amminolinker steht) (0,1mM) mit sulfo-KMUS (5mM) in Kopplungspuffer (50mM Na PO₄ und 75mM NaCl, 0,5x, pH 7.6) bei 37°C für 2h aktiviert. Die Reaktion wurde mit 50 mM Tris-(Hydroxymethyl)-Aminomethane (pH 7,5) gestoppt und das aktivierte ODN nach Ethanolpräzipation (300mM NaOAc pH 5.2, 5.5 mM MgCl₂, 100% Ethanol), Zentrifugation und einmaligem Waschen mit 70% Ethanol erhalten. Das so erhaltene ODN (0,1mM) wurde in Kopplungspuffer (50mM NaPO₄ und 75mM NaCl, 0,5x, pH7.0) gelöst und reagierte mit dem Peptid (0,2mM) für eine Stunde bei 37°C. Die Reaktion wurde durch ein denaturierendes Polyacrylamidgel (20%) und Ethidiumbromidfärbung überprüft. Das entstandene NLS gekoppelte ODN wurde durch HPLC aufgereinigt und zur Synthese der zirkulären Expressionsvektoren verwendet.

### Beispiel 2b: Herstellung der zirkulären Vektoren mit Peptidkopplung

Das Plasmid pMOK-Luc wurde mit dem Restriktionsenzym Eco31I für 2h bei 37°C vollständig verdaut. Durch den Restriktionsverdau wurden zwei DNA-Fragmente erhalten. Mittels des Enzymes T4-DNA-Ligase wurden die zuvor bei 90°C für 3min hybridisierten, komplementären, 5' phosphorylierten Oligodesoxynukleotide (TIBMolBiol, Berlin) 5'-PH- GGGAACCTTCAGTxAGCAATGG-3' und 5' PH- AGGGCCATTGCTxACTGAAGG-3' (xT steht für amminomodifizierte Thyminbase mit C2 Amminolinker, an welches wahlweise über ein Crosslinkermolekül das Signalpeptid NLS kovalent gebunden wurde) in Anwesenheit des Restriktionsenzymes Eco31l über Nacht bei 4°C an das den Vektor bildende DNA Fragment (vgl. Beispiel 1) ligiert. Das resultierende Gemisch an Nukleinsäuren wurde mit dem Enzym T7-DNA-Polymerase behandelt. Das Produkt wurde durch Anionenaustauschchromatographie aufgereinigt und mit Isopropanol gefällt.

### Beispiel 3: Transfektion der Zellen, Expressionsnachweis

Zellen der K562 Zelllinie wurden mit dem Plasmid pMOK-Luc, einem linearen Vektor und dem erfindungsgemäßen Vektor mit Ligandenkopplung durch Elektroporation transfiziert. Die Durchführung erfolgte im Dreifach-Ansatz, wobei nach vorheriger Konzentrationsbestimmung jeweils 100ng DNA eingesetzt wurden. Je Ansatz wurden 2,5x10⁸Zellen / 250µl verwendet, welche bei 300V und 1050µF transfiziert wurden. Nach einer Inkubation von 24h bei 37°C erfolgte der Nachweis der Expression über die Luciferaseaktivitätsbestimmung im Luminumeter. Die Ergebnisse sind in Figur 3 dargestellt.

### Beispiel 4: Transfektion mit Lipofektin

In 24-Loch-Platten wurden am Tag vor der Transfektion 40.000 Zellen der humanen Zelllinie Hela ausgesät (20.000 Zellen/cm²). Die Zellen wurden unter Verwendung von Lipofektin mit folgender DNA transfiziert: Plasmid pMOK, linearer Vektor, zirkulärer Vektor ohne Peptid-Kopplung; jeweils kodierend für das Reportergen Luciferase. Die Durchführung erfolgte im 4-8-fach Ansatz, wobei nach vorheriger Konzentrationsbestimmung jeweils 800ng DNA eingesetzt wurden. Die DNA wurde mit dem Transfektionsreagent Lipofektin in DMEM (Dulbecco's Modified Eagle Medium) 45' bei 20°C inkubiert. Anschließend erfolgte Transfektion durch die Zugabe des DNA-Lipofektin-Gemisches zu den Zellen. Die Inkubationsdauer betrug 4h bei 37°C. Nach einem Mediumwechsel wurden die Zellen 21 h bei 37°C weiter kultiviert, nachfolgend schloß sich der Nachweis der Expression über die Luciferaseaktivitätsbestimmung im Luminumeter an. Die Ergebnisse sind in Figur 4 dargestellt.

### Beispiel 5: intratumorale Injektion von Lac-Z kodierenden Vektoren

Drei Gruppen zu je 6 Mäusen wurden intratumoral mittels eines Jet-Injektion-Verfahrens linearer Vektor und zirkulärer Vektor mit und ohne Peptidkopplung kodierend für das Lac-Z Gen injiziert. Die Tiere bekamen insgesamt 5 Injektionen. Die DNA-Konzentration betrug jeweils 1 µg/µl. 48h nach der letzten Injektion wurden die Tiere getötet, der Tumor entnommen und für weitere Untersuchungen in flüssigem Stickstoff aufbewahrt. Die Präparation der Tumorzellen erfolgte durch Homogenisierung in 800µl Lysispuffer (TE-Puffer, pH 8, enthaltend Aprotinin 10mg/ml und PMSF 10µg/ml). Nach Zentrifugation (14000rpm, 4°C, 10min), wurde das Lysat erhalten und die Proteinbestimmung mit Hilfe eines Coomassie Tests (Pierce, Rockford, USA) durchgeführt. Die Absorption wurde bei 595 nm bestimmt. Die Erbebnisse sind in Fig. 5 gezeigt.

### Beispiel 6: Interferon-gamma Sekretion nach Immunisierung mit HBsAg

6-8 Wochen alte BALB/c Mäuse wurden i.d. mit für HBsAg kodierenden Vektoren (gelöst in 50µl 100mM Na₂PO₄) immunisiert. Nach 4 Wochen wurden von je 2 Mäusen pro Gruppe die Milz erhalten und die Splenozyten isoliert. Die Splenozyten wurden mit Concanavalin A (ConA), mit Mytomycin C behandelten Antigenpräsentierende Zellen (APC, als Negativkontrolle) und mit APC's, die mit dem HBsAg-Peptid (Positivkontrolle) Kontakt hatten über Nacht bei 37°C unter 5% CO₂ inkubiert. Die 96-Loch Platten wurden zuvor mit 8µg/ml Anti-Maus IFN-gamma Antikörper (Pharmingen) beschichtet. Nach der Inkubation wurden die Zellen entfernt und 100µl biotinylierter Anti-Maus IFN-gamma Antikörper (Pharmingen) der Konzentration von 2µg/ml auf die 96-Loch Platten gegeben. Nach der Inkubation über Nacht bei 4°C wurden die Platten gewaschen und nach Zugabe von 100µl einer 1:800 Verdünnung von Avidin-Peroxidase (Pharmingen) 1 h bei Raumtemperatur inkubiert. Durch Zugabe von 100µl DAB Substrat (Sigma) erfolgte die Entwicklung. Nach 20min wurde die Reaktion gestoppt und die Präzipitate mit Hilfe eines Stereomikroskopes gezählt. Die Ergebnisse der Negativkontrolle wurden von den Ergebnissen der Positivkontrolle abgezogen und so die Zahl der antigenspezifischen Präzipitate ermittelt. Diese Werte wurden ins Verhältnis mit den Ergebnissen der mit ConA inkubierten Splenozyten gesetzt. Die Ergebnisse sind in Figur 6 dargestellt.

## Patentansprüche

1. Verfahren zur Herstellung eines zirkulären, ringförmig geschlossenen Expressionskonstrukts aus einem DNA-Doppelstrang, bestehend aus den folgenden Schritten:
a) Ausschneiden einer doppelsträngigen DNA-Sequenz durch einen primären Verdau mit Restriktionsendonukleasen aus einem in pro- oder eukaryonten Zellen amplifizierbaren Plasmid,
b) wobei die Schnittstellen die Sequenzen einer Expressionskassette, bestehend aus
i. wenigstens einer Promotorsequenz,
ii. wenigstens einer kodierenden Sequenz, und
iii. wenigstens einer Polyadenylierungssequenz,
direkt, ohne dazwischen liegende Basen, an beiden Seiten begrenzen, und
c) anschließender intramolekularer Ligation der entstandenen Restriktionsfragmente, so dass bei der Ligationsreaktion ein kovalent geschlossener DNA-Doppelstrang entsteht (Ringschluss), gefolgt von
d) einem sekundären Verdau des Reaktionsgemisches mit einer Restriktionsendonuklease, die eine auf dem herzustellenden Expressionskonstrukt nicht vorhandene, auf dem Rest des biologisch amplifizierbaren Plasmids jedoch wenigstens einmal vorkommende Erkennungssequenz erkennt und schneidet, und
e) gleichzeitigem oder anschließendem Abbau des offenkettigen Rests des biologisch vermehrbaren Plasmids mit einer für 3'- und 5'-DNA-Enden spezifischen Exonuklease und
f) der Aufreinigung der ringförmig geschlossenen Expressionskassette aus einem DNA-Doppelstrang.

2. Verfahren zur Herstellung eines zirkulären, ringförmig geschlossenen Expressionskonstrukts aus einem DNA-Doppelstrang, bestehend aus den folgenden Schritten:
a) Ausschneiden einer doppelsträngigen DNA-Sequenz durch einen primären Verdau mit Restriktionsendonukleasen aus einem in pro- oder eukaryonten Zellen amplifizierbaren Plasmid,
b) wobei die Schnittstellen die Sequenzen einer Expressionskassette, bestehend aus
i. wenigstens einer Promotorsequenz,
ii. wenigstens einer kodierenden Sequenz, und
iii. wenigstens einer Polyadenylierungssequenz,
direkt, ohne dazwischen liegende Basen, an beiden Seiten begrenzen, und
c) anschließender intramolekularer Ligation der entstandenen Restriktionsfragmente in Anwesenheit wenigstens eines Oligodesoxynukleotids, an welches kovalent mittels chemischer Modifikation wenigstens ein Ligand gebunden ist, so dass bei der Ligationsreaktion ein kovalent geschlossener DNA-Doppelstrang entsteht (Ringschluss) unter Einschluss des Oligodesoxynukleotids, gefolgt von
d) einem sekundären Verdau des Reaktionsgemisches mit einer Restriktionsendonuklease, die eine auf dem herzustellenden Expressionskonstrukt nicht vorhandene, auf dem Rest des biologisch amplifizierbaren Plasmids jedoch wenigstens einmal vorkommende Erkennungssequenz erkennt und schneidet, und
e) gleichzeitigem oder anschließendem Abbau des offenkettigen Rests des biologisch vermehrbaren Plasmids mit einer für 3'- und 5'-DNA-Enden spezifischen Exonuklease und
f) der Aufreinigung der ringförmig geschlossenen Expressionskassette aus einem DNA-Doppelstrang.

3. Verfahren nach Anspruch 2, wobei das Oligodesoxynukleotid durch eine oder mehrere alkylische Carbonsäure-, Amin-, Thiol- oder Aldehydfunktionen chemisch modifiziert wird.

4. Verfahren nach Anspruch 1 oder 2, wobei der primäre Restriktionsverdau mittels einer oder mehrerer Restriktionsendonukleasen des Typs IIS, vorzugsweise Eco31I, durchgeführt wird.

5. Verfahren nach Anspruch 1 oder 2, wobei der sekundäre Restriktionsverdau vorzugsweise mit dem Enzym Eco1471 durchgeführt wird.

6. Expressionskonstrukt zum Transport genetischer Information, bestehend aus doppelsträngiger DNA, wobei
a) das Expressionskonstrukt zirkulär, ringförmig geschlossen ist und auf die zur Expression notwendigen Sequenzinformationen reduziert ist, ferner
b) das Expressionskonstrukt nicht in pro- oder eukaryonten Zellen amplifizierbar ist, sowie
c) das Expressionskonstrukt zumindest aus wenigstens einer Expressionskassette aus doppelsträngiger DNA besteht, und wobei eine Expressionskassette
i. wenigstens eine Promotorsequenz,
ii. wenigstens eine kodierende Sequenz, und
iii. wenigstens eine Polyadenylierungssequenz enthält,
d) das Expressionskonstrukt 200 bis 10.000 bp umfasst.

7. Expressionskonstrukt nach Anspruch 6, wobei das Expressionskonstrukt wenigstens bevorzugt 1000-2.500 bp umfasst.

8. Expressionskonstrukt nach Anspruch 6 oder 7, wobei das Expressionskonstrukt wenigstens ein Oligodesoxynukleotid enthält.

9. Expressionskonstrukt nach Anspruch 8, wobei das Oligodesoxynukleotid mindestens eine aminomodifizierte Thyminbase aufweist.

10. Expressionskonstrukt nach Anspruch 8 oder 9, wobei das Oligodesoxynukleotid durch eine oder mehrere alkylische Carbonsäure-, Amin-, Thiol- oder Aldehydfunktionen chemisch modifizierbar ist.

11. Expressionskonstrukt nach den Ansprüchen 6 bis 10, wobei an das Oligodesoxynukleotid wenigstens ein Ligand kovalent gebunden ist.

12. Expressionskonstrukt nach Anspruch 11, wobei der Ligand ein Oligopeptid ist.

13. Expressionskonstrukt nach Anspruch 12, wobei das Oligopeptid aus 3 bis 30 Aminosäuren besteht, wobei es sich mindestens zur Hälfte um die basischen Aminosäuren Arginin und/oder Lysin handelt.

14. Expressionskonstrukt Anspruch 12, wobei das Oligopeptid eine Kernlokalisationssequenz der Aminosäuresequenz PKKKRKV aufweist.

15. Verwendung des Expressionskonstrukts entsprechend einem oder mehrerer der vorhergehenden Ansprüche zur Herstellung von Vakzinen.

16. Verwendung des Expressionskonstrukts entsprechend einem oder mehrerer der vorhergehenden Ansprüche als Bestandteil eines Kits.

## Claims

1. Method for the production of a circular, annular closed expression construct from a DNA double strand, comprising the following steps:
a) Cleavage of a double stranded DNA sequence by a primary digestion with restriction endonucleases from an in pro- or eukaryotic cells amplifiable plasmid,
b) where the recognition sites limit the sequences of an expression cassette comprising
i. at least one promotor sequence,
ii. at least one coding sequence, and
iii. at least one poly-adenylation sequence,
directly, without any in-between located bases, on both sites, and
c) subsequent intramolecular ligation of the produced restriction fragments, so that a covalently closed DNA double strand develops (annulated closing) from the ligation reaction, followed by
d) a secondary digestion of the restriction mixture with a restriction endonuclease cutting a recognition sequence not present on the expression construct to be produced, but at least once present on the rest of the biological amplifiable plasmid, and
e) concurrent or following degradation of the unclosed rest of the biological amplifiable plasmid with an exonuclease specific for 3'- and 5'-DNA ends and
f) purification of the annular closed expression cassette from a DNA double strand.

2. Method for the production of a circular, annular closed expression construct from a DNA double strand, comprising the following steps:
a) Cleavage of a double stranded DNA sequence by a primary digestion with restriction endonucleases from an in pro- or eukaryotic cells amplifiable plasmid,
b) where the recognition sites limit the sequences of an expression cassette comprising
i. at least one promotor sequence,
ii. at least one coding sequence, and
iii. at least one poly-adenylation sequence,
directly, without any in-between located bases, on both sites, and
c) subsequent intramolecular ligation of the produced restriction fragments in the presence of at least one oligodeoxynucleotide to that at least one ligand is bound covalently via chemical modifications, so that a covalently closed DNA double strand develops (annulated closing) under incorporation of the oligodeoxynucleotide, followed by
d) a secondary digestion of the restriction mixture with a restriction endonuclease cutting a recognition sequence not present on the expression construct to be produced, but at least once present on the rest of the biological amplifiable plasmid, and
e) concurrent or following degradation of the unclosed rest of the biological amplifiable plasmid with an exonuclease specific for 3'-and 5'-DNA ends and
f) purification of the annular closed expression cassette from a DNA double strand.

3. Method according to claim 2, where the oligodeoxynucleotide is chemical modified by one or more carbonic acid, amine, thiol, or aldehyde function.

4. Method according to claim 1 or 2, where the primary restriction digestion is done by one or more type IIS restriction endonucleases, preferably Eco31I.

5. Method according to claim 1 or 2, where the secondary restriction digestion is done preferably with the enzyme Eco1471.

6. Expression construct for the transport of genetic information, comprising double stranded DNA, where
a) the expression construct is circular, annular closed and reduced to the sequence information necessary for expression, further
b) the expression construct is not amplifiable in pro- or eukaryotic cells, as well as
c) the expression construct consists at least of an expression cassette of double stranded DNA, and where a expression cassette comprises
i. at least one promotor sequence,
ii. at least one coding sequence, and
iii. at least one polyadenylation sequence,
d) the expression construct spans 200 to 10 000 bp.

7. Expression construct according to claim 6, where the expression construct spans at least 1000 to 2500 bp.

8. Expression construct according to claim 6 or 7, where the expression construct comprises at least one oligodeoxynucleotide.

9. Expression construct according to claim 8, where the oligodeoxynucleotide has at least one amino-modified thymine-base.

10. Expression construct according to claim 8 or 9, where the oligodeoxynucleotide is chemical modifiable by one or more carbonic acid, amine, thiol or aldehyde functions.

11. Expression construct according to the claims 6 to 10, wheat at least one ligand is covalently bound to the oligodeoxynucleotide

12. Expression construct according to claim 11, where the ligand is an oligopeptide.

13. Expression construct according to claim 12, where the oligo-peptide consists of 3 to 30 amino acids, where at least half are the basic amino acids arginine and/or lysine.

14. Expression construct according to claim 12, where the oligo-peptide has a nucleus localisation sequence with amino acid sequence PKKKRKV.

15. Use of the expression construct according to one or more of the previous claims for the production of a vaccine.

16. Use of the expression construct according to one or more of the previous claims as part of a kit

## Revendications

1. Procédé de préparation ou de fabrication d'un vecteur d'expression circulaire fermé en forme d'anneau à partir d'un ADN bicaténaire, composé des étapes suivantes :
a) découpage d'une séquence d'ADN bicaténaire par une digestion primaire avec des endonucléases de restriction issues d'un plasmide amplifiable dans des cellules pro- ou eucaryotes,
b) les sites de coupure délimitant au niveau des deux côtés directement, sans bases placées entre, les séquences d'une cassette d'expression, composée de
i. au moins une séquence promoteur,
ii. au moins une séquence codante, et
iii. au moins une séquence de polyadénylation,
et
c) ligature intramoléculaire subséquente des fragments de restriction formés, de façon telle que lors de la réaction de ligature, il se forme un double brin d'ADN fermé de façon covalente (boucle), suivie de
d) digestion secondaire du mélange réactionnel avec une endonucléase de restriction qui reconnaît et coupe une séquence de reconnaissance non présente sur le vecteur d'expression à fabriquer mais cependant existante au moins une fois sur le radical du plasmide biologiquement amplifiable, et
e) dégradation simultanée ou consécutive du radical à chaîne ouverte du plasmide biologiquement multipliable avec une exonucléase spécifique des extrémités d'ADN 3' et 5', et
f) purification de la cassette d'expression fermée en forme d'anneau formée d'un ADN bicaténaire.

2. Procédé de préparation d'un vecteur d'expression circulaire fermé en en forme d'anneau à partir d'un ADN bicaténaire, composé des étapes suivantes :
a) découpage d'une séquence d'ADN bicaténaire par une digestion primaire avec des endonucléases de restriction issues d'un plasmide amplifiable dans des cellules pro- ou eucaryotes,
b) les sites de coupure délimitant au niveau des deux côtés directement, sans bases placées entre, les séquences d'une cassette d'expression, composée de
i. au moins une séquence promoteur,
ii. au moins une séquence codante, et
iii. au moins une séquence de polyadénylation,
et
c) ligature intramoléculaire subséquente des fragments de restriction formés en présence d'au moins un oligodésoxynucléotide auquel est lié de façon covalente, au moyen d'une modification chimique, au moins un ligand, de façon telle que lors de la réaction de ligature, il se forme un double brin d'ADN fermé de façon covalente (boucle) par incorporation de l'oligodésoxynucléotide, suivie de
d) digestion secondaire du mélange réactionnel avec une endonucléase de restriction qui reconnaît et coupe une séquence de reconnaissance non présente sur le vecteur d'expression à fabriquer mais cependant présente au moins une fois sur le radical du plasmide biologiquement amplifiable, et
e) dégradation simultanée ou consécutive du radical à chaîne ouverte du plasmide biologiquement multipliable avec une exonucléase spécifique des extrémités d'ADN 3' et 5', et
f) purification de la cassette d'expression fermée en forme d'anneau formée d'un ADN bicaténaire.

3. Procédé selon la revendication 2, dans lequel l'oligodésoxynucléotide est modifié chimiquement par une ou plusieurs fonctions alkyliques d'acide carboxylique, d'amine, de thiol ou d'aldéhyde.

4. Procédé selon la revendication 1 ou 2, dans lequel la digestion de restriction primaire est conduite au moyen d'une ou de plusieurs endonucléases de restriction du type IIS, de préférence Eco 31I.

5. Procédé selon la revendication 1 ou 2, dans lequel la digestion de restriction secondaire est conduite de préférence avec l'enzyme Eco147I.

6. Vecteur d'expression destiné au transport d'informations génétiques, composé d'un ADN bicaténaire, dans lequel
a) le vecteur d'expression est circulaire, fermé en forme d'anneau, et est réduit aux informations de séquence nécessaires à l'expression, et
b) le vecteur d'expression n'est pas amplifiable dans des cellules pro- ou eucaryotes,
c) le vecteur d'expression se composant d'au moins une cassette d'expression d'ADN bicaténaire, et dans lequel une cassette d'expression contient
i. au moins une séquence promoteur,
ii. au moins une séquence codante, et
iii. au moins une séquence de polyadénylation,
d) le vecteur d'expression comporte 200 à 10 000 pb.

7. Vecteur d'expression selon la revendication 6, dans lequel le vecteur d'expression comprend au moins de préférence 1 000 à 2 500 pb.

8. Vecteur d'expression selon la revendication 6 ou 7, dans lequel le vecteur d'expression contient au moins un oligodésoxynucléotide.

9. Vecteur d'expression selon la revendication 8, dans lequel l'oligodésoxynucléotide présente au moins une base thymine amino-modifiée.

10. Vecteur d'expression selon la revendication 8 ou 9, dans lequel l'oligodésoxynucléotide peut être chimiquement modifié par une ou plusieurs fonctions alkyliques d'acide carboxylique, d'amine, de thiol ou d'aldéhyde.

11. Vecteur d'expression selon les revendications 6 à 10, dans lequel au moins un ligand est lié de façon covalente à l'oligodésoxynucléotide.

12. Vecteur d'expression selon la revendication 11, dans lequel le ligand est un oligopeptide.

13. Vecteur d'expression selon la revendication 12, dans lequel l'oligopeptide se compose de 3 à 30 acides aminés, au moins la moitié des acides aminés étant de l'arginine et/ou de la lysine basiques.

14. Vecteur d'expression selon la revendication 12, dans lequel l'oligopeptide présente une séquence de localisation nucléaire de la séquence d'acides aminés PKKKRKV.

15. Utilisation du vecteur d'expression selon une ou plusieurs des revendications précédentes pour fabriquer des vaccins.

16. Utilisation du vecteur d'expression selon une ou plusieurs des revendications précédentes en tant que composant d'une trousse ou d'un ensemble.
